# EUROPEAN PATENT APPLICATION

(11) **EP 4 785 904 A2**
(43) Date of publication of application: **05.08.2026**
(21) Application number: 26185623.1
(22) Date of filing: 17.01.2023
(51) Int. Cl.: A61F 2/08

(54) **COMPRESSED IMPLANTS FOR SOFT TISSUE REPAIR**

(30) Priority: 17.01.2022 US 202263300162 P
(62) Divisional of application: 23740908.1
(71) Applicant: Embody, Inc., Norfolk, VA 23508 (US)
(72) Inventor: MAGHDOURI-WHITE, Yas, Norfolk, VA (US); BROWN, Robert S., Norfolk, 23508 (US); HAVENER, Matthew, Norfolk, 23508 (US); CHRISTENSEN, Kyle W., Norfolk, 23508 (US)
(74) Representative: Aldridge, Henry Alexander

(57) **Abstract**

An implant comprising one or more biopolymer sheets with compressed portions and uncompressed portions is disclosed. The compressed portions have a decreased thickness and increased density compared to uncompressed portions of the implant. This contributes to a modified flexibility for the compressed portions compared to the uncompressed portions. The compressed portions can include one or more portions or one or more edges of the implant, including substantially the entire periphery of the implant. The compressed edges help manage the "deflection" of the periphery that can occur in the highpressure fluid environment used to establish the operating space at the site of repair of damaged tissue.

## Description

### CROSS-REFERENCE TO RELATED APPLICATIONS

This application claims the benefit of Provisional Patent Application Number 63/300,162 filed January 17, 2022, and titled "Compressed Implants for Soft Tissue Repair," which is incorporated by reference herein in its entirety.

### BACKGROUND

Generally, implantable scaffolds are used in the operating room or other acute care settings and may be cut and shaped to fit and support a given soft tissue site. Scaffolds may be placed in proximity to or in contact with tissue that has torn and been repaired, for example with sutures, suture anchors or surgical glue. Scaffolds provide support and reinforcement of soft tissues, such as tendons and ligaments, including the Achilles tendons, rotator cuffs, patellar tendons, biceps tendons, and quadriceps tendons. The scaffold shares some of the mechanical stress and load with the repaired tissue. Preferably, such scaffolds provide a suitable matrix for tissue growth. Scaffolds may be packaged in sterile containers either individually or in pairs or in larger quantities.

During arthroscopic surgery, for example, to repair a shoulder, pressurized fluid may be used to enhance visibility at the surgical site and to provide physical space for implanting and affixing a scaffold to support or repair damaged or injured tissue. However, for relatively flexible scaffolds, the pressurized fluid can make insertion, control and placement of the scaffold more difficult.

### SUMMARY

In one aspect, an implant, including one or more biopolymer sheets; the implant having a first portion and a second portion; where the first portion has a first thickness; where the second portion has a second thickness that is less than the first thickness; and where a portion of the implant having the second thickness is present at one or more edges of the implant.

In some embodiments, the first portion has a first density, the second portion has a second density, and the second density is greater than the first density.

In some embodiments, the biopolymer sheet has a nonwoven construction.

In some embodiments, the biopolymer sheet is comprised of collagen fibers and optionally with fibers comprising collagen and bioacceptable polymers.

In some embodiments, the first portion of the biopolymer sheet is uncompressed; and the second portion of the biopolymer sheet is compressed.

In some embodiments, the second thickness is less than about 10% of the first thickness.

In some embodiments, the second portion includes a compressed peripheral portion of the biopolymer sheet.

In some embodiments, the sheet has a sheet width; and a peripheral edge portion has a peripheral width, wherein the peripheral width is between about 1% and 13% of the sheet width.

In some embodiments, the second portion has a second portion surface area on the first side of the implant, and the second portion surface area is between about 0.5% and 50% of the total implant surface area on the first side.

In some embodiments, the second portion includes at least one corner of the biopolymer sheet.

In some embodiments, the second portion forms a boundary about a periphery of the biopolymer sheet.

In some embodiments, the biopolymer sheet includes a compressed interior portion that separates two or more uncompressed portions of the biopolymer sheet.

In some embodiments, the compressed interior portion is substantially X-shaped.

In some embodiments, the compressed interior portion is configured as a plurality of substantially parallel lines.

In some embodiments, the substantially parallel lines are substantially parallel with aligned fibers of the biopolymer sheet.

In some embodiments, the second portion forms a border about the periphery of the biopolymer sheet; and at least one portion of the second portion has a curved geometry.

In some embodiments, the at least one portion with the curved geometry is oriented in a direction that is substantially parallel to aligned fibers of the biopolymer sheet.

In some embodiments, the second portion is less flexible than the first portion.

In some embodiments, the second portion extends around an outer periphery of the implant, and the lesser flexibility of the second portion relative to the first portion reduces deflection of the outer periphery of the implant.

In some embodiments, the first portion is uncompressed, the second portion is compressed, and the second portion better resists delamination of the implant when the implant is subjected to fluid pressure compared to the first portion.

In some embodiments, the first portion is uncompressed, the second portion is compressed, and the second portion has improved dye retention compared to the first portion.

In some embodiments, the first portion is uncompressed, the second portion is compressed, and the second portion better resists tearing than the first portion.

In another aspect, a method of making an implant includes steps of: providing a biopolymer sheet having an initial sheet thickness; placing a stencil against the biopolymer sheet, the stencil covering select portions of the biopolymer sheet; and applying a load to press the stencil against the biopolymer sheet to create a modified biopolymer sheet, where the modified biopolymer sheet includes at least one uncompressed portion having the initial sheet thickness and at least one compressed portion having a compressed thickness.

In some embodiments, the compressed thickness of the at least one compressed portion is between about 0.5% and 10% of the initial sheet thickness.

In some embodiments, the compression process is performed at approximately room temperature.

In some embodiments, the biopolymer sheet has a nonwoven construction.

In some embodiments, the biopolymer sheet is formed of high strength collagen fibers.

In some embodiments, the at least one compressed portion is less flexible than the at least one uncompressed portion.

In some embodiments, the biopolymer sheet has an initial flexibility prior to the step of applying the load to the stencil against the polymer sheet, and the modified biopolymer sheet has a final flexibility after the step of applying the load to the stencil against the polymer sheet, wherein the final flexibility is less than the initial flexibility.

In some embodiments, the modified biopolymer sheet includes a first uncompressed portion and a second uncompressed portion separated by the at least one compressed portion, and the method further includes cutting the biopolymer sheet along the at least one compressed portion to create a first sheet-like implant and a second sheet-like implant.

In some embodiments, cutting includes laser cutting the biopolymer sheet along the at least one compressed portion.

In another aspect, a method of supporting the repair of soft tissue, using an implant, the implant comprising a biopolymer sheet including selectively compressed portions and uncompressed portions, includes steps of: implanting the implant into a surgical site including a pressurized fluid environment within a body of a patient; manipulating the implant to position the implant; and securing the implant to tissue at the surgical site.

In some embodiments, the implanting the implant further includes inserting the implant to the surgical site through a cannula while the implant is in a collapsed configuration.

In some embodiments, the method includes delivering the implant beyond a distal end of the cannula so that the implant returns to an un-collapsed configuration after being inserted into the body through the cannula.

In some embodiments, the method further includes applying a dye to the implant.

In some embodiments, the dye is retained for a longer period on the compressed portions of the implant than on the uncompressed portions of the implant within the pressurized fluid environment.

In some embodiments, the compressed interior portion configured as a plurality of substantially parallel lines is dyed.

Other systems, methods, features and advantages of the embodiments will be, or will become, apparent to one of ordinary skill in the art upon examination of the following figures and detailed description. It is intended that all such additional systems, methods, features and advantages be included within this description and this summary, be within the scope of the embodiments, and be protected by the following claims.

### BRIEF DESCRIPTION OF THE DRAWINGS

The embodiments can be better understood with reference to the following drawings and description. The components in the figures are not necessarily to scale, with emphasis instead being placed upon illustrating the principles of the embodiments. Moreover, in the figures, like reference numerals designate corresponding parts throughout the different views.
FIG. 1 is a schematic view of a portion of a biopolymer sheet, according to an embodiment;
FIG. 2 is a schematic view of an implant comprised of a biopolymer sheet with compressed edges, according to an embodiment;
FIG. 3 is a schematic side view of an implant comprised of a biopolymer sheet with compressed edges, according to an embodiment;
FIG. 4 is a schematic side view of an implant comprised of a biopolymer sheet with compressed edges, according to another embodiment;
FIG. 5 is a schematic enlarged view of a compressed edge of an implant, according to an embodiment;
FIGS. 6-11 are schematic views of multiple embodiments of implants having compressed portions configured with different geometries;
FIG. 12 is a schematic view of a method for making implants with compressed edges, according to an embodiment;
FIGS. 13-16 are schematic views of steps in a method of making implants with compressed edges, according to an embodiment;
FIGS. 17 and 18 are schematic views showing an implant with compressed edges that includes a transition area where the thickness changes gradually, according to an embodiment; and
FIGS. 19A-C are various schematic views of implants with compressed edges operating in dynamic fluid environments.

### DETAILED DESCRIPTION

### Definitions

Various terms that are used throughout the detailed description and in the claims are collected here for reference.

"Biopolymer" means a naturally occurring, protein-based macromolecule natively found in connective and other soft tissue and in the extracellular matrix, such as collagen, fibrin, fibrinogen, gelatin, and laminin.

"Bio-compatible polymer" means a synthetic polymer capable of being mixed or blended with a biopolymer to add various desirable properties, for example, strength or rigidity as would otherwise be provided by the biopolymer alone.

"High-strength collagen fiber" means a fiber comprising collagen with an ultimate tensile strength of at least 80 MPa.

"Scaffold" means a construct formed from biopolymers and/or copolymers. A scaffold could comprise a linear structure, such as a suture, a two-dimensional structure, such as a patch, ribbon, or sheet, as well as any suitable three-dimensional structure. In some embodiments, such constructs are substantially aligned fibers formed into layers, mats, sheets and tubes.

"Substantially aligned fibers" means that approximately at least half of the fibers lying within 15 to 20 degrees of a reference in a scaffold are oriented along a common axis. This is to be interpreted in contrast to randomly oriented fibers.

A "compressed" portion of an implant, scaffold, component, or material layer, refers to a portion that has been pressed, squeezed, molded or crimped, under a mechanical load. The compression process reduces the thickness and increases the density of the compressed portion. Compression may also reduce fiber to fiber movement. Thus, compared to an otherwise similar "uncompressed" portion of an implant, a compressed portion may be substantially thinner and have a higher density than the uncompressed portion. The higher density is a result of compressing a given quantity of material into a smaller volume. Moreover, the increased density generally reduces the flexibility of the compressed portions, compared to the flexibility of the uncompressed portions. Moreover, an implant with compressed portions will generally be less flexible, overall, than its pre-compressed configuration, even as it retains some uncompressed portions.

### Biopolymer Sheets

The embodiments provide implantable biocompatible scaffolds in the form of surgical implants, and surgical and orthopedic devices utilizing such scaffolds, preferably including sheet-like or pad-like structures, as well as related methods for their production and use to support the repair of injured soft and hard tissues, and to stabilize and support various body structures including ligaments, tendons, and joints. For example, the implants can be used in surgeries for tendons and ligaments including, but not limited to the Achilles tendon, rotator cuff, patellar tendon, biceps tendon, quadriceps tendon, gluteus medius, and plantar plate. Thus, implants according to the invention may be applied and used by medical personnel in pressurized as well as non-pressurized repair sites.

In a preferred embodiment, the scaffold (or implant) comprises a sheet-like or pad-like construct, in which the scaffold is further comprised of biopolymer fibers, preferably formed of collagen or mixtures of collagen and bioacceptable (or biodegradable) polymers, preferably PLLA, PDLA, PDLLA, PLGA, poly(glycolic acid), and mixtures thereof. A preferred mixture of polymers in a scaffold for supporting the repair of soft tissue injury in a human or other mammal, comprises about 10 to 40% by weight of Type I collagen and about 60 to 90% by weight of such a bioacceptable copolymer.

In such embodiments, the term "sheet" or "biopolymer sheet" may be used to refer to the scaffold. Optionally, the scaffold may be formed of multiple individual sheets of different compositions and porosity that are held together by conventional means, for example, such as laser tacking, stitching, or suturing.

The scaffold is designed to be resorbed over time and replaced with the patient's own tissue during the course of the healing process. The implantable biocompatible scaffold is designed to promote healing in the tissue (such as ligaments, tendons or other suitable tissues) and to support accelerated return to activity by enabling relatively sooner physical therapy as compared to conventional alternative recovery and rehabilitative treatments.

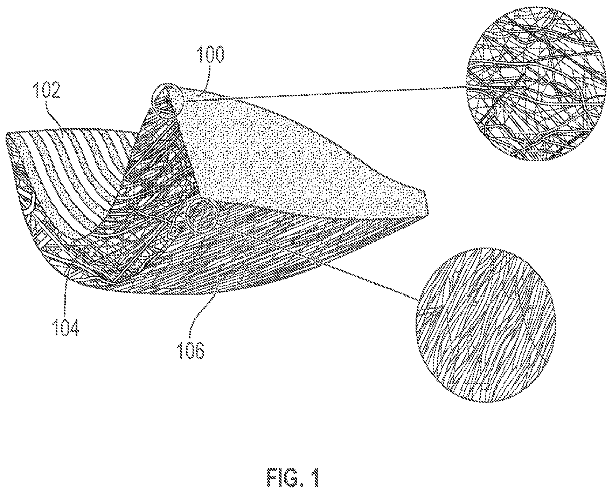

As an example, FIG. 1 shows a biopolymer sheet 100 (sheet 100), which is comprised of collagen fibers. In some embodiments, an outer surface 102 of sheet 100 is comprised of unaligned fibers designed for isotropic suture retention, strength, and structural integrity. By contrast, the bulk of sheet 100 (bulk interior 104) and interior surface 106 may be comprised of substantially aligned fibers having a porous microfiber and consistent microarchitecture. Sheet 100 may be constructed so that it will be completely absorbed during the remodeling process as it promotes tissue ingrowth.

The biopolymer sheets of the embodiments preferably have a nonwoven construction. Persons skilled in the art will understand that implants having woven as well as non-woven components may be utilized in the devices and methods of the present invention.

Implants according to the present invention can be comprised of any suitable biopolymer compositions and blends of biopolymers and, optionally, with bio-compatible polymers. In some embodiments, sheets may be constructed from electrospun blends of Poly-d, I-lactic acid (PDLLA) with Type I collagen. Preferred examples are described in U.S. Patent Number 10,617,787, to Francis et al., entitled "Biopolymer Compositions, Scaffolds and Devices," the entirety which is herein incorporated by reference. Additionally, as additional preferred examples, implants and collagen fibers can be produced using any of the methods and components described in U.S. Patent Number 11,213,610, to Francis et al., entitled "Biopolymer Scaffold Implants and Methods for their Production," U.S. Patent Number 11,020,509 to Francis et al., entitled "Microfluidic Extrusion," and U.S. Published Application 2021/0275716 to Francis et al., entitled "Braided Surgical Implants," the entireties of which are herein incorporated by reference.

The embodiments provide an implant with features that help solve various technical problems that might be encountered when trying to manipulate the implant in pressurized fluid environments. These problems include unwanted deflection (including bending, twisting and folding) at the edges of the implant; sheet delamination (or separation); dye retention; and possible tearing.

To help overcome at least these problems, the embodiments provide biopolymer sheets that include both compressed portions and uncompressed portions. As used herein, a compressed portion of a sheet is a portion of the sheet that has been pressed or squeezed along its thickness, relative to any uncompressed portions of the sheet. The compressed portions are, relative to uncompressed portions of the sheet, thinner, denser, less flexible, and less prone to tearing. In some embodiments, formation of the compressed portions is achieved by applying a mechanical load to an uncompressed area of a biopolymer sheet.

The implants of the embodiments can therefore be characterized as having at least two different portions (or regions) with substantially different thicknesses. Similarly, the implants of the embodiments can be characterized as having at least two different portions (or regions) with substantially different densities.

### Sheets with Compressed Portions

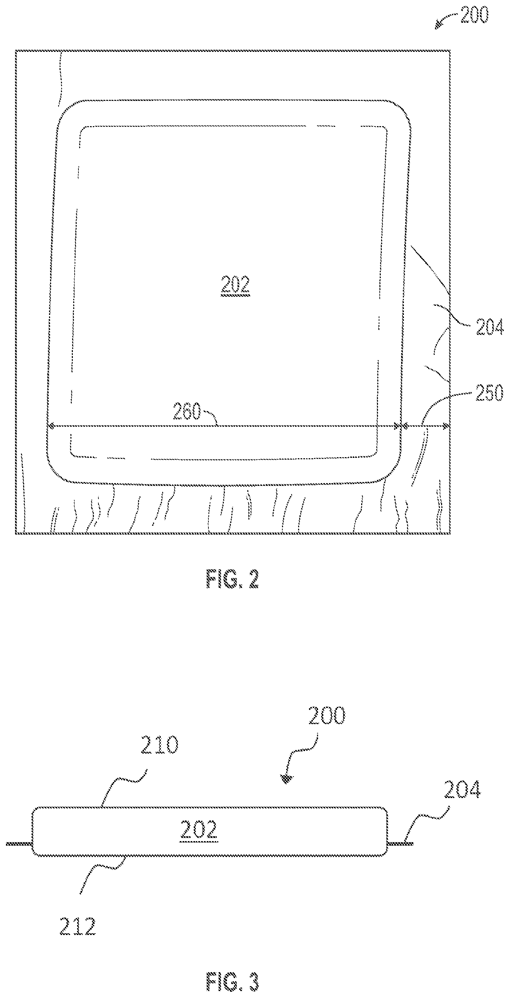

FIGS. 2 and 3 illustrate a bottom view and a side view, respectively, of an implant 200. Implant 200 is comprised of a biopolymer sheet that includes an uncompressed interior portion 202 and a compressed peripheral portion 204. It may be appreciated that uncompressed interior portion 202 and compressed peripheral portion 204 are comprised of the same continuous biopolymer sheet, such that at least some fibers within uncompressed interior portion 202 extend through to compressed peripheral portion 204.

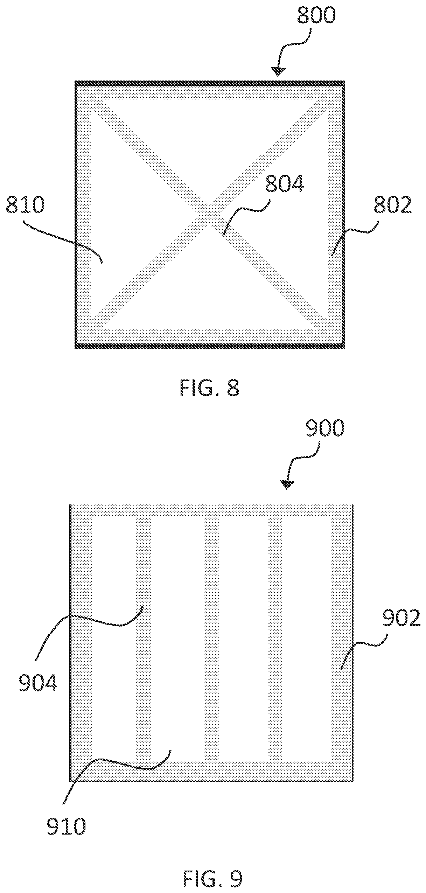

In this exemplary embodiment, peripheral portion 204 completely surrounds uncompressed interior portion 202, thereby providing compressed outer edges for implant 200. Put another way, the entire periphery of implant 200 is compressed. In other embodiments, however, the periphery of implant 200 could be comprised of both compressed and uncompressed portions (see, for example, FIGS. 8 and 9).

In FIGS. 2-3, implant 200 has a puffy, or pillow-like shape, with uncompressed interior portion 202 extends, or "bulges," away from a planar surface coincident with compressed peripheral portion 204. More specifically, uncompressed interior portion 202 extends outwardly along both inner side 210 and outer side 212, with compressed peripheral portion 204 lying approximately in a plane between the outer and inner sides of implant 200.

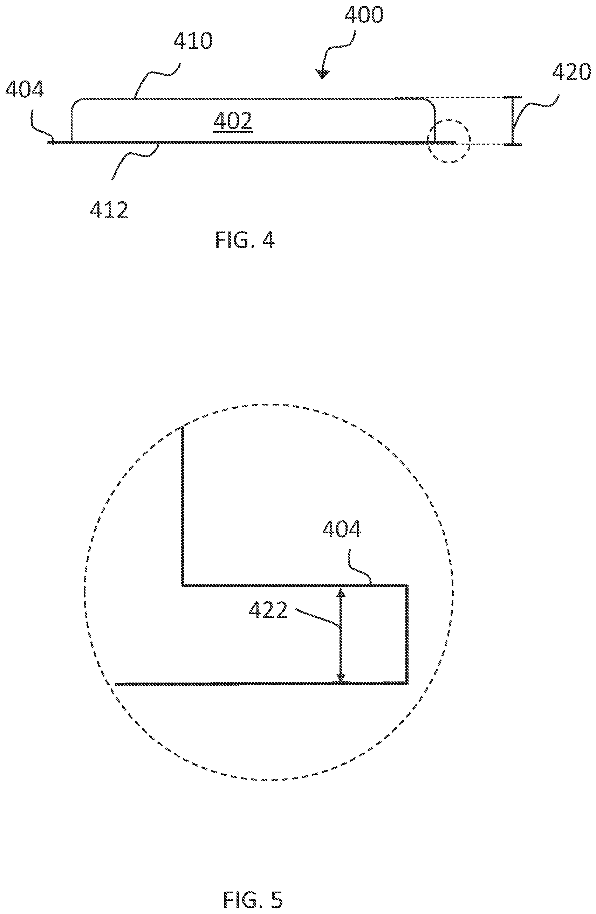

In another embodiment, one side of an implant could be relatively flat so that the compressed peripheral portion is approximately planar with the relatively flat side. Referring to FIG. 4, an implant 400 has a "ravioli-like" shape. In this case, uncompressed interior portion 402 is relatively flat on outer side 412. Moreover, compressed peripheral portion 404 lies approximately in the same plane as uncompressed interior portion 402 on outer side 412. By contrast, uncompressed interior portion 402 bulges out on inner side 410. More specifically, uncompressed interior portion 402 bulges out from the planar surface defined by uncompressed interior portion 402 and compressed peripheral portion 404 on outer side 412.

In other embodiments, implants with compressed portions could have other suitable geometries including quilted or paneled geometries. As another example, an implant with compressed edges could have a trapezoidal geometry.

### Utility of Compressed Portions

As already mentioned, the use of an implant composed of one or more biopolymer sheets having compressed portions, preferably including compressed edges, will address and at least substantially resolve various technical problems that are commonly encountered when implanting such scaffolds in environments characterized by the presence and use of pressurized fluids.

For example, when inserting a flexible sheet-like implant during procedures at a surgical site in the presence of a pressurized fluid, such as saline, that is characterized by having a dynamic fluid flow, a sheet-like implant without compressed portions may deflect at its edges, thereby making it more difficult to correctly place, align, and/or secure the implant. In the exemplary embodiments, compressed portions of the implant help control deflection by reducing the relative flexibility of the implant along the edges. This reduced flexibility may be due, in part, to the increased density of the implant within the compressed portions. As discussed in further detail below, the flexibility may also be affected by the overall shape and size (for example, the width and thickness) of the compressed portions. The reduced flexibility helps provide a more stable configuration for the implant. This reduces the tendency of the implant to bend, fold, twist and/or flutter under dynamic fluid flow, thereby allowing for easier placement, alignment, and fixation of the implant within a surgical site in the body of a human or other animal requiring such surgical treatment.

Because the (uncompressed) biopolymer sheets of the embodiments tend to have a high porosity, dye or coloration that is optionally applied to the sheets, for example to mark the edges and/or to indicate proper alignment for implantation, may be partially washed away by fluids present at the repair site during a surgical procedure, making visibility of the sheet relatively more difficult for the entire duration of the procedure. The compressed portions of the exemplary implants, by contrast, have relatively higher density, and thus may improve dye retention by modifying fluid flow through the compressed portions, compared to uncompressed portions. For example, if the peripheral edges of a dyed implant are compressed, then the dye may be retained longer on the peripheral edge, allowing the surgeon improved visibility of the edges of the implant through the entire procedure.

Using compressed portions along some, or optionally substantially all, of the periphery of a biopolymer sheet may also reduce delamination or separation of fibers that can occur when the edges of a biopolymer sheet are subject to dynamic fluid flow. In particular, within the compressed portions of an implant, contact between biopolymer fibers may be increased relative to fibers in uncompressed portions of an implant. Furthermore, movement between fibers may be reduced in the compressed portions compared to uncompressed portions of an implant. This tighter packing and reduced movement of adjacent fibers at the edges of the implant may limit fluid intrusion into the edges, thereby eliminating or slowing the process of fiber separation.

Because compressed portions may have an increased fiber to fiber contact and decreased fiber separation as compared to uncompressed portions, compressed portions may also reduce accidental tearing, and/or prevent any tears once present from spreading or propagating to the edges of an implant. In some cases, compressed portions may have increased suture or anchor retention compared to uncompressed portions, since the rigidity and/or strength of the compressed portions may be greater relative to uncompressed portions.

Compressed edges may also present a reduced frontal area for fluid flow compared to uncompressed portions of an implant. The reduced frontal area may help improve placement of the implant at the surgical repair site where it ultimately is affixed, as the edges are subjected to fewer fluid forces that might tend to push, bend, or otherwise interfere with placement of the implant.

The degree to which compressed portions can limit the flexibility of an implant, reduce separation, enhance dye retention, and/or limit/mitigate tearing may depend on the relative dimensions of the compressed portions to the dimensions of the uncompressed portions. These dimensions include the relative thicknesses, relative widths (and/or lengths), and relative areas for the compressed and uncompressed portions.

### Relative Thickness

The relative thickness between a compressed portion and an uncompressed portion of an implant comprised of a biopolymer sheet may be best understood with reference to FIG. 4 as well as to FIG. 5, which shows an enlarged view of a portion of implant 400. Referring to both FIGS. 4 and 5, uncompressed interior portion 402 has an uncompressed thickness 420 (shown in FIG. 4) while compressed peripheral portion 404 has a compressed thickness 422 (shown in FIG. 5). For purposes of illustration, it may be appreciated that some of these dimensions are not shown to scale.

In different embodiments, the relative thicknesses of compressed and uncompressed portions can be varied. Changing the relative thickness of the compressed and uncompressed portions may result in a change in relative flexibility between the compressed and uncompressed portions and may also vary the overall flexibility of the implant as a whole. In some embodiments, compressed thickness 422 may be approximately between 0.5% and 25% of the original uncompressed thickness. In other embodiments, compressed thickness 422 may be approximately between 1% and 3% of uncompressed thickness 420. In some embodiments, for example, the value of compressed thickness 422 may be approximately 95% less than the value of uncompressed thickness 420. In other embodiments, the value of compressed thickness may be about 60, 65, 70, 75, 80, 85, 90, 95 or 99% less than the value of uncompressed thickness 420.

For reference, exemplary measurements of thickness for compressed and uncompressed portions are provided herein. It may be appreciated, however, that different absolute thicknesses could be used while still providing relative thicknesses in the ranges discussed above. Referring to FIG. 4, in some embodiments, uncompressed thickness 420 could have a value approximately in a range between 2-10 mm. In other embodiments, uncompressed thickness 420 could have a value approximately in a range between 2-6 mm. In still other embodiments, uncompressed thickness 420 could have a value approximately in a range between 3-5 mm.

Referring to FIG. 5, in some embodiments, compressed thickness 422 could have a value approximately less than 1 mm. In some embodiments, compressed thickness 422 could have a value approximately in a range between 20-1000µm (0.02-1 mm). In other embodiments, compressed thickness 422 could have a value approximately in a range between 200-800µm (0.2 - 0.8 mm).

### Peripheral Width

A compressed peripheral portion can be characterized by the width of one or more edges. Referring to FIG. 2, for example, compressed peripheral portion 204 may have a peripheral width 250. In this case, peripheral width 250 is the width of one of the four peripheral edge portions that together comprise compressed peripheral portion 204. When the compressed portion extends around the entire periphery of the implant (as in FIG. 2), the peripheral width may be referred to as a "border width".

Uncompressed peripheral portion 202 can have an interior width 260. In non-square configurations, peripheral portion 202 could have a different interior length as well.

In a "wide edge" embodiment, peripheral width 250 may have a value approximately in the range between about 2.5-4 mm. For an implant with overall dimensions of about 30x30 mm, as an example, this corresponds to an interior width of about 22-24 mm. This also corresponds to a peripheral width that is about 10% to 13% of the total implant width. In another, "narrow edge" embodiment, peripheral width 250 may have a value approximately in the range between about 0.5-2.5 mm. For a 30x30 mm implant, this corresponds to an interior width of about 26-29 mm. This also corresponds to a peripheral width that is about 1% to 7% of the total implant width.

### Flexibility

As already discussed, using compressed edges may reduce the flexibility of an implant, which reduces the tendency of the implant to bend, fold, and stick to itself during arthroscopic implantation. As one measure of this reduced flexibility, implants can be hydrated and draped over an object such as a glass rod to determine how compressed and uncompressed (or non-compressed) portions may tend to deform relative to one another. A specific measure may include an angle of bending, or "draped angle" between two sides of a portion of an implant as it is draped over a glass rod or similar component. A smaller draped angle indicates more bending and flexibility around a rod compared to a larger draped angle. In particular, a draped angle close to zero would indicate that the portion of implant has been nearly folded in half around the rod, while a draped angle close to one hundred and eighty degrees would indicate that the portion of implant has not substantially bent or folded around the rod.

In some embodiments, the compressed portions of an implant may tend to have sufficiently larger draped angles than uncompressed portions, owing to the reduced flexibility of the compressed portions compared to the uncompressed portions. That is, the compressed portions may provide greater resistance to bending around the rod compared to uncompressed portions. In some embodiments, the uncompressed portions may have a draped angle approximately in a range between twenty and forty degrees. In some embodiments, the compressed portions may have a draped angle approximately in a range between forty and ninety degrees. In some embodiments, a compressed portion may have a draped angle that is at least ten degrees less than the draped angle of an uncompressed portion. In some embodiments, a compressed portion may have a draped angle that is at least twenty degrees less than the draped angle of an uncompressed portion. In some embodiments, a compressed portion may have a draped angle that is at least twenty-five degrees less than the draped angle of an uncompressed portion. By sizing each implant so that the implant is slightly larger than the physical area to be treated, the compressed portions may add sufficient rigidity at the edges of the implant for easier insertion and placement at the repair or treatment site, while the uncompressed portion interior to the edges provides drapability at the implantation site where the implant approximates or contacts that site.

### Variations Along Periphery

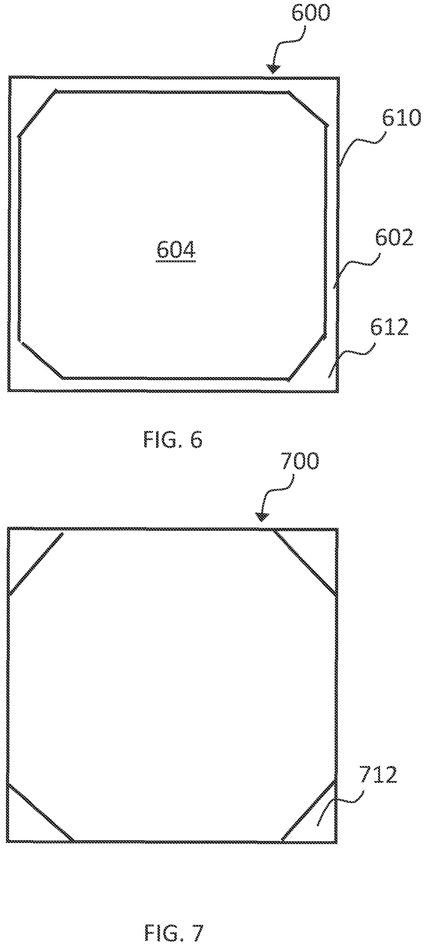

The properties of an implant can also be modified by adjusting the locations and shapes of compressed portions. For example, the embodiment of FIG. 6 shows a configuration in which an implant 600 has a compressed peripheral portion 602 and an uncompressed interior portion 604. The compressed peripheral portion 602 has a narrow width along the edges 610 of implant 600, and also includes compressed corners 612 that are triangular in shape and extend further into the interior of implant 600 to provide additional reinforcement at the corners. In an exemplary embodiment, compressed peripheral portion 602 is comprised of a border with a width in a range between about 0.5-2.5 mm. Each compressed triangular corner may have a hypotenuse in a range between about 2-6 mm. Using compressed corners with narrow compressed edges, as in FIG. 6, may help maximize the uncompressed area of the implant while preventing delamination and improving dye retention along the entire periphery of the implant.

In another embodiment, shown in FIG. 7, only the corners 712 of implant 700 may be compressed. Using only compressed corners may help maximize the uncompressed area of the implant while preventing delamination and improving dye retention at the corners of the implant.

### Variations Along Interior

FIGS. 8 and 9 illustrate two embodiments of an implant in which the compressed portions of the implant extend through an interior region of the implant, as well as around the periphery. For example, in FIG. 8, an implant 800 includes a compressed outer peripheral portion 802 and a compressed interior portion 804. In this example, compressed interior portion 804 has an X-like geometry, with two narrow compressed lines that extend from opposing corners and intersect at a center of the implant.

Compressed interior portion 804 has the effect of dividing the interior of the implant into four separate uncompressed portions 810. In this case, each of the uncompressed portions 810 have an approximately triangular shape. In an exemplary embodiment, compressed outer peripheral portion 802 has a border width approximately in a range between about 0.5-2 mm. Also, the lines forming the "X" of the compressed interior portion 804 may also each have a width approximately in a range between about 0.5-2 mm.

In the embodiment shown in FIG. 9, an implant 900 includes a compressed outer peripheral portion 902 and a compressed interior portion 904. In this example, compressed interior portion 904 is configured as a set of parallel lines. In some embodiments, the parallel lines may be oriented along the direction of fiber alignment in the biopolymer sheet.

Compressed interior portion 904 has the effect of dividing the interior of the implant into four separate uncompressed portions 910. In this case, each of the uncompressed portions 910 have an approximately rectangular shape. In an exemplary embodiment, compressed outer peripheral portion 902 has a thin border and compressed interior portion 904 includes lines or strips, each with a width approximately in a range between about 0.5-2 mm. In some cases, the spacing between adjacent lines of compressed interior portion 904 may be approximately in a range between about 4-6 mm.

Using compressed portions within the interior of an implant can help in reducing unwanted deflection of the interior of the implant, reduce delamination through the interior, and improve die retention through the interior. Additionally, using compressed portions to create separated uncompressed portions within the interior (for example, uncompressed portions 810 and uncompressed portions 910), may help mitigate tearing. Specifically, if a tear forms in one of the uncompressed portions, it may be less likely to spread to other, separated, uncompressed portions, thereby limiting the chance that a tear can extend throughout the whole implant.

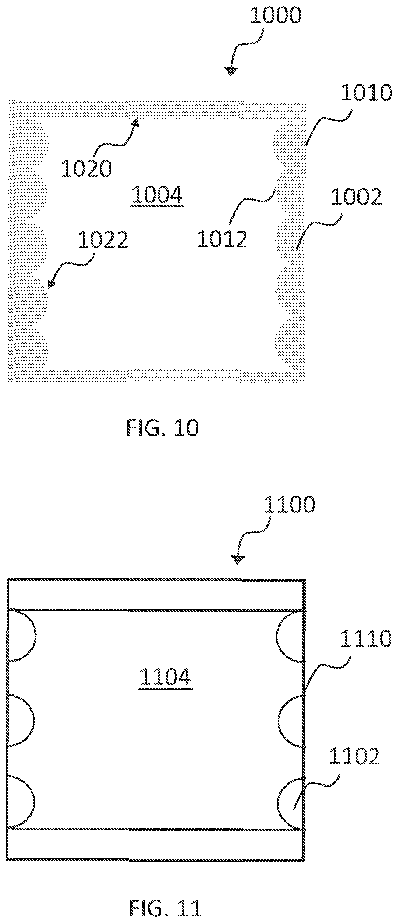

Some embodiments can include compressed portions with curved or otherwise irregular boundaries. In FIG. 10, an implant 1000 as a compressed peripheral portion 1002 and an uncompressed interior portion 1004. In this case, an outer boundary 1010 of compressed peripheral portion 1002 has a rectangular shape. However, an inner boundary 1012 has different shapes along different edges. Specifically, a first set of opposing edges 1020 are straight along inner boundary 1012, while a second set of opposing edges 1022 are substantially curved along inner boundary 1012. In particular, a second set of opposing edges 1022 may have a "scalloped" shape. By scalloping the inner edge of compressed peripheral portion 1002, this configuration may reduce the amount of stress and shearing in the transition area between the compressed and uncompressed portions. Moreover, in the embodiment of FIG. 10, the orientation of the scalloped edges may run in parallel with the substantially aligned fibers of the implant. This configuration may help reduce the propagation of longitudinal or transverse tears..

Other embodiments could use other shapes along an inner or outer boundary of a compressed portion. Other exemplary shapes could include, but are not limited to: waves, zig-zags, dentil edges, and/or irregular curves or various radii.

FIG. 11 shows an alternative embodiment for an implant 1100. In this example, the uncompressed interior portion 1104 extends to the outermost edge 1110 of implant 1100 in some areas. These uncompressed areas are separated by distinct compressed peripheral portions 1102. This configuration may facilitate reduced stress and shearing in the transition area, as with the embodiment of FIG. 10, while also increasing the surface area of the uncompressed portion, relative to the embodiment of FIG. 10.

The relative areas of the compressed and uncompressed portions can vary, according to the shape and size of the compressed and uncompressed portions. Depending on the presence and width of the border, the presence, shape and size of interior portions, and dimensions of the biopolymer sheet, the relative surface area of the compressed portions (along one side of the implant) may vary between about 0.1% and 50% of the total implant area, preferably about 0.2%, 0.3%, 0.4%, 0.5%, 0.6%, 0.7%, 0.8%, 0.9%, 1.0%, 1.25%, 1.5%, 1.75%, 2.0%, 2.25%, 2.5%, 2.75%, 3.0%, 3.5%, 4.0%, 4.5%, 5.0%, 7.5%, 10%, 15%, 20%, 25%, 30%, 35%, 40% and 45% of the total implant area.

By varying the relative thicknesses, relative widths, and relative areas of the compressed portions to uncompressed portions, as well as adjusting the placement and shape of compressed portions, the implant can be tuned to achieve desired properties for flexibility, delamination, dye retention, and tear mitigation. For example, generally increasing the relative width and/or relative area of compressed portions to uncompressed portions provides decreased flexibility for the implant relative to a completely uncompressed implant of the same shape and dimensions. This minimizes deflection of the implant during surgery to facilitate easier positioning, alignment, and attachment of the implant to the target tissue (for example, a tendon).

### Method of Making

The embodiments provide methods for compressing biopolymer sheets to create implants with compressed and uncompressed portions. In some embodiments, selective portions of a biopolymer sheet can be compressed using a stencil. The stencil could comprise a material with openings. When pressed against the biopolymer sheets, the solid portions of the stencil press down on the underlying portions of the biopolymer sheet to create compressed portions. The openings in the stencils correspond to regions where the biopolymer sheet is not be pressed, and therefore such portions would remain uncompressed after the load has been removed.

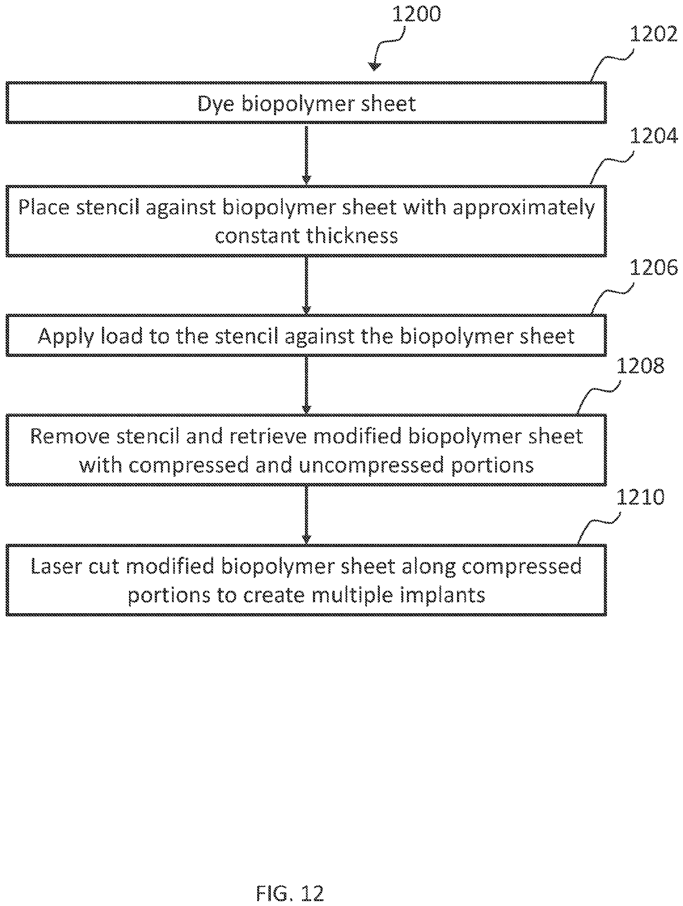

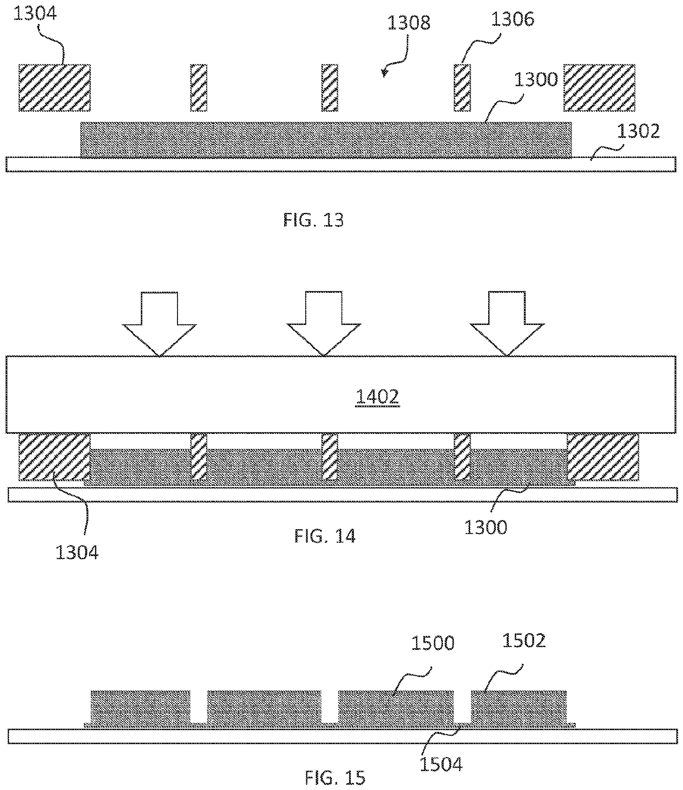

FIG. 12 illustrates a schematic method 1200 for forming implants by modifying biopolymer sheets. Some steps of the method are shown in FIGS. 13-15.

In step 1202, the biopolymer sheet may be dyed. As discussed above, dying the sheet can facilitate increased visualization of the implant during the surgical procedure. Any suitable methods for applying dye to the biopolymer sheet could be used.

Next, in step 1204, a stencil may be placed against a biopolymer sheet. Stencils may be made of any suitable materials including soft or hard plastics, or other suitable materials. Some specific examples of materials that could be used for stencils include polylactide (PLA) and silicon cord. Some embodiments could use additional layers of rubber and/or thin ultrahigh molecular weight polyethylene (UHMWPE) sheets to facilitate more uniform compression. Here, the entirety of the provided biopolymer sheet may be in an uncompressed state and may have an approximately constant thickness (also referred to as the "initial thickness" of the biopolymer sheet). FIG. 13 shows a schematic view of an uncompressed biopolymer sheet 1300 placed on a supporting surface 1302. A stencil 1304 is shown in cross-section. The stencil is seen to include solid material portions 1306 that act to press down on the biopolymer sheet, as well as openings 1308 that prevent the underlying portions of the biopolymer sheet from being pressed. Although shown in cross-section, it may be appreciated that in the exemplary embodiment the openings have a square or approximately rectangular geometry. In other embodiments, openings in a stencil could have any suitable geometry. In step 1206, a load may be applied to the stencil against the biopolymer sheet. In the example shown in FIG. 14, a press 1402 is used to apply a downward force against stencil 1304. This causes stencil 1304 to compress biopolymer sheet 1300 at select portions. In one embodiment, a mechanical arm is used to press down on the stencil. The applied load may be sufficient to achieve the desired compression, but not so high that the fibers of the biopolymer sheet are damaged. In one exemplary embodiment, approximately fifty pounds of load may be applied to achieve the desired level of compression. Persons skilled in the art will adjust the pressure in order to produce desired amounts of compression of the implant.

For clarity, only a supporting surface 1302 and stencil 1304 are shown for facilitating compression of biopolymer sheet 1300. However, in some embodiments layers of UHMWPE and rubber can be used on either side of the biopolymer sheet to add cushioning to facilitate uniform compression. In one embodiment, the biopolymer sheet and stencil may be sandwiched between two cushioning assemblies. Each cushioning assembly may be further comprised of a sheet of rubber (approximately 1/8^{th} inch in thickness) sandwiched between two thin sheets of UHMWPE (approximately 0.25 inches in thickness). That is, one cushioning assembly (a sheet of rubber sandwiched between sheets of UHMWPE) may be placed on a supporting surface, and then the biopolymer sheet laid on top of that assembly. The stencil would be laid on the biopolymer sheet, and then another cushioning assembly (a sheet of rubber sandwiched between sheets of UHMWPE) would be placed over the stencil. Then a press would be applied.

In step 1208, the stencil is removed and the resulting modified biopolymer sheet (modified sheet 1500 in FIG. 15) can be retrieved. The resulting sheet has both compressed and uncompressed portions. The resulting modified sheet will include both compressed and uncompressed portions. As an example, FIG. 15 shows an embodiment of a modified sheet 1500 with compressed portions 1502 and uncompressed portions 1504.

In step 1210, the modified biopolymer sheet can be cut along the compressed portions to create multiple implants having both compressed and uncompressed portions. In some cases, laser cutting can be used.

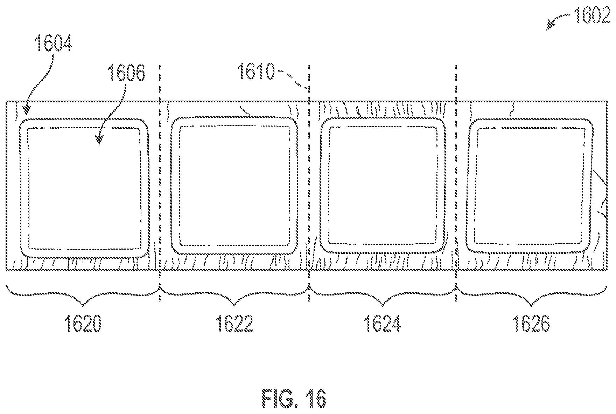

As an example, FIG. 16 is a schematic view of a modified biopolymer sheet 1602 that includes both compressed portions 1604 and uncompressed portions 1606. Cut lines 1610 indicate locations where biopolymer sheet 1602 may be cut, thereby separating the sheet into four separate implants (implant 1620, implant 1622, implant 1624, and implant 1626). The final implants may be similar to one another, with each implant including a compressed peripheral portion and an uncompressed interior portion.

Thus, the embodiments provide an efficient process for manufacturing implants with compressed and uncompressed portions that minimizes damage to the fibers that could be caused by heat or the application of other substances. Moreover, because cutting occurs along compressed portions, the resulting cut edges may be "cleaner" or more regular, compared to cuts that might be made through uncompressed portions.

As already discussed, implants manufactured in this way may help provide better control of deflection, improved dye retention and visualization during surgery, reduced delamination of edges and/or corners, and reduced tearing and/or propagation of existing tears.

### Transition Thickness

For clarity, the embodiments of FIGS. 12-15 depict a configuration using stencils with generally vertical opening sidewalls. The resulting geometry for the implant may be one like that depicted in FIG. 5, in which the thickness of the implant changes abruptly at the transition area between the compressed portion and the uncompressed portion. In some embodiments, the manufacturing process could be modified to produce implants where the thickness changes more gradually in this transition zone. This alternative geometry may help reduce (or distribute) stresses at these transition areas.

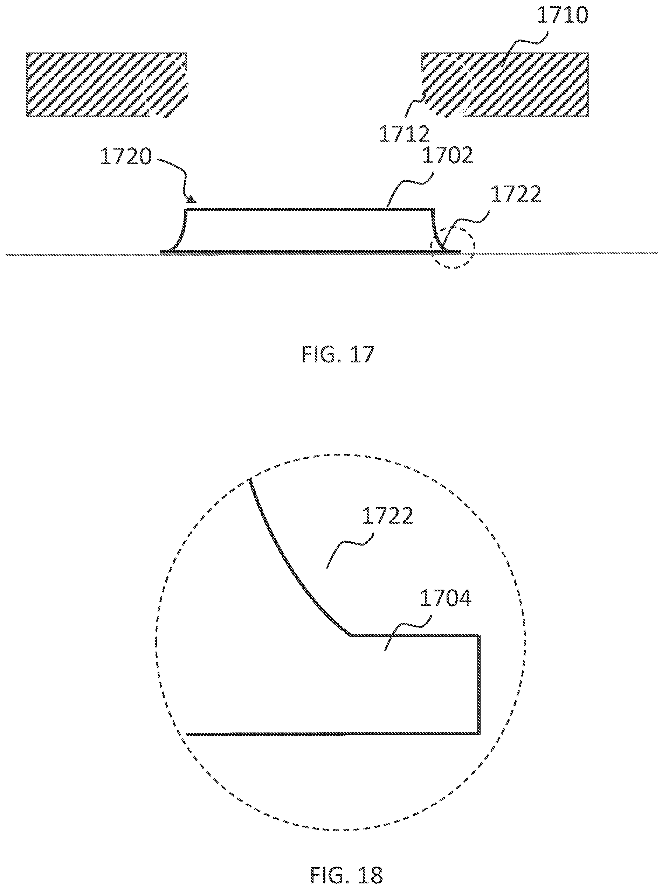

As one example, FIG. 17 shows an embodiment in which a stencil 1710 has curved interior opening walls 1712. In this embodiment, the opening walls 1712 have a convex curvature resulting in a concave geometry for the transition area 1722 of an implant 1720. Moreover, as seen in FIG. 18, which shows an enlarged view of the transition area, this concave geometry corresponds to a more gradual change in the thickness of implant 1710 between the fully compressed peripheral portion 1704 and the uncompressed portion 1702 (see FIG. 17). The resulting implant may have regions with a fully uncompressed thickness, regions with a fully compressed thickness, and a variable thickness within the transition area.

In other embodiments, the curvature of the transition area of an implant could be convex, rather than concave. Such a geometry could be achieved by using a stencil with interior opening walls that have a concave curvature.

### Results Under Dynamic Fluid Flow

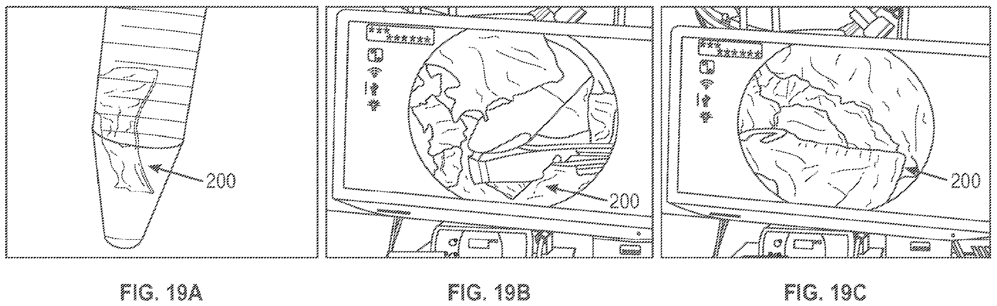

FIGS. 19A-C show how a biopolymer sheet with compressed portions can help overcome various technical problems discussed above in the context of dynamic fluid flow. In particular, FIG. 19A shows a portion of a biopolymer sheet with a compressed edge following vigorous shaking in fluid within a test tube. As seen in FIG. 19A, the compressed edge does not show signs of delamination.

Likewise, FIGS. 19B-C show an implant with compressed edges being successfully placed and anchored down in an arthroscopic implantation in a shoulder under fluid pressure of 25-50 mmHg. FIGS. 19B-C also show how the compressed edges of the implant better retain dye compared to the uncompressed interior of the implant, thereby improving visualization through the entirety of the procedure over sheets with no compressed portions.

### Method of Use

The implant can be used to support repair of soft tissue. In one embodiment, the implant can be collapsed and inserted through a cannula into an operating space within the body. Such techniques are described, for example, in published U.S. Published Application Serial Number 2021/0275220, entitled "Surgical Cannula with Removable Pressure Seal," the contents of which are expressly incorporated by reference in their entirety. In some cases, the implant is inserted in a pressurized fluid environment. Once the implant passes through a distal end of the cannula, the implant returns to an un-collapsed configuration. At this point, a surgeon can manipulate the implant to position the implant over the surgical sight (which may comprise a tendon to be repaired, for example). Finally, the surgeon can secure the implant as appropriate at the surgical site. For example, an implant can be secured with sutures, staples, anchors and other fasteners known to persons skilled in the art.

Using an implant with compressed portions, as discussed above, may make visibility and manipulation of the implant easier, by reducing deflection of the edges under fluid flow and by increasing the amount of time that a dye remains visible on the implant. Compressed edges can also reduce the tendency of the implant to separate or delaminate into layers under fluid pressure, thereby maintaining a discrete edge that the surgeon can visualize and manipulate.

While various embodiments have been described, the description is intended to be exemplary, rather than limiting and it will be apparent to those of ordinary skill in the art that many more embodiments and implementations are possible that are within the scope of the embodiments. Although many possible combinations of features are shown in the accompanying figures and discussed in this detailed description, many other combinations of the disclosed features are possible. Any feature of any embodiment may be used in combination with or substituted for any other feature or element in any other embodiment unless specifically restricted. Therefore, it will be understood that any of the features shown and/or discussed in the present disclosure may be implemented together in any suitable combination. Accordingly, the embodiments are not to be restricted except in light of the attached claims and their equivalents. Also, various modifications and changes may be made within the scope of the attached claims.

## Claims

1. An implant (200), comprising:
one biopolymer sheet having a non-woven construction and including fibers comprised of a mixture of collagen and one or more bioacceptable polymers,
wherein an outer surface of the one biopolymer sheet is comprised of the fibers arranged in an unaligned orientation, and
wherein a bulk of the one biopolymer sheet and an interior surface of the one biopolymer sheet are comprised of the fibers arranged in a substantially aligned orientation.

2. The implant of claim 1, wherein the one or more bioacceptable polymers comprises Poly-d, I-lactic acid (PDLLA).

3. The implant of any of claims 1 or 2, wherein the one or more bioacceptable polymers comprises PDLA.

4. The implant of claim 2, wherein the one biopolymer sheet is constructed from an electrospun blend of collagen and PDLLA.

5. The implant of claim 1, wherein the mixture of collagen and one or more bioacceptable polymers comprises 10-40% by weight Type I collagen and 60-90% by weight bioacceptable copolymer.

6. The implant (200) of claim 1, wherein the one biopolymer sheet has a first portion (202) and a second portion (204), the first portion (202) being uncompressed and having a first thickness, the second portion (204) being compressed and having a second thickness that is less than the first thickness.

7. The implant (200) of claim 6, wherein a portion of the one biopolymer sheet having the second thickness is present at one or more edges of the one biopolymer sheet.

8. The implant (200) of claim 1, wherein the first portion (202) has a first density, wherein the second portion (204) has a second density, and wherein the second density is greater than the first density.

9. The implant (200) of claim 1, wherein the second thickness is less than about 10% of the first thickness;
optionally wherein the one biopolymer sheet has a sheet width, wherein a peripheral edge portion of the one biopolymer sheet has a peripheral width (250), wherein the peripheral width is between about 1% and 13% of the sheet width.

10. The implant (200) of claim 1, wherein the second portion (204) includes a compressed peripheral portion of the one biopolymer sheet; or
wherein the second portion (204) has a second portion surface area on a first side of the implant (200), and wherein the second portion surface area is between about 0.5% and 50% of a total implant surface area on the first side of the implant (200).

11. The implant (200) of claim 1, wherein the second portion (204) includes at least one corner of the one biopolymer sheet;
optionally wherein the second portion (204) forms a boundary about a periphery of the one biopolymer sheet.

12. The implant (200) of claim 1, wherein the one biopolymer sheet includes a compressed interior portion that separates two or more uncompressed portions of the one biopolymer sheet;
optionally wherein:
the compressed interior portion is substantially X-shaped; or
the compressed interior portion is configured as a plurality of substantially parallel lines, further optionally wherein the substantially parallel lines are substantially parallel with the fibers arranged in the substantially aligned orientation, or wherein the compressed interior portion configured as a plurality of substantially parallel lines is dyed.

13. The implant (200) of claim 1, wherein the second portion (204) forms a border about the periphery of the one biopolymer sheet; and
wherein at least one portion of the second portion (204) has a curved geometry;
optionally wherein the at least one portion with the curved geometry is oriented in a direction that is substantially parallel to the fibers arranged in the substantially aligned orientation.

14. The implant (200) of claim 1, wherein the second portion (204) is less flexible than the first portion (202);
optionally wherein the second portion (204) extends around an outer periphery of the implant (200), and wherein the lesser flexibility of the second portion (204) relative to the first portion (202) reduces deflection of the outer periphery of the implant.

15. The implant (200) of claim 1, wherein:
the second portion (204) better resists delamination of the implant (200) when the implant (200) is subjected to fluid pressure compared to the first portion (202); or
the second portion (204) has improved dye retention compared to the first portion (202); or.
the second portion (204) better resists tearing than the first portion (202).
